# EUROPÄISCHE PATENTSCHRIFT

(11) **EP 0 995 794 B1**
(45) Veröffentlichungstag und Bekanntmachung des Hinweises auf die Patenterteilung: **24.04.2002**
(21) Anmeldenummer: 99114674.7
(22) Anmeldetag: 27.07.1999
(51) Int. Cl.: C12M 1/16, C12M 1/18, C12M 1/32, C12M 1/34

(54) **Trägersystem für ein Nährmedium und Nährboden sowie Verfahren zu deren Herstellung**
Carrier system for a culture medium and culture medium and process for their production
Système de porteur pour un milieu de culture et milieu de culture et procédé de leur production

(30) Priorität: 30.07.1998 DE 19834427
(43) Veröffentlichungstag der Anmeldung: 26.04.2000
(73) Patentinhaber: VITAKRAFT-WERKE, Wührmann & Sohn, 28307 Bremen (DE)
(72) Erfinder: Gröbe, Anneliese, Dr., 79106 Freiburg (DE); Bremer, Heinz, Prof. Dr., 40468 Düsseldorf (DE)
(74) Vertreter: Leissler-Gerstl, Gabriele

(56) Entgegenhaltungen:
- EP-A- 0 398 703
- DE-A- 3 128 415
- GB-A- 2 233 760

## Beschreibung

Die Erfindung betrifft ein Trägersystem für ein Nährmedium, einen Nährboden gemäß dem Oberbegriff von Anspruch 16 sowie Verfahren zur Herstellung des Trägersystems und des Nährbodens.

Nährböden werden allgemein in der Biochemie zum Züchten von Mikroorganismen eingesetzt. Ein neueres Anwendungsgebiet für solche Nährböden ist die Aquaristik, wo der Wunsch besteht, die Population an Mikroorganismen im Wasser von Aquarien zu untersuchen. Hierzu wurden Nährböden entwickelt, die neben einem Trägersystem und einem Nährmedium einen Farbindikator aufweisen, mit dem die in Kolonien gezüchteten Mikroorganismen angefärbt und somit nachgewiesen und gegebenenfalls auch ausgezählt werden können. Das Wachstum der gezüchteten Mikroorganismen wird durch Inkubieren bei einer bestimmen Temperatur über eine bestimmte Zeit erhöht und gleichzeitig auf eine definierte Basis gestellt.

Nährböden arbeiten entweder nach dem Abklatschprinzip oder nach dem Benetzungsprinzip. Im ersten Fall (Abklatschprinzip) wird der Nährboden auf eine zu prüfende Fläche angedrückt, wodurch die Mikroorganismen auf die Oberfläche des Nährbodens gelangen und dort durch das Nährmedium zum Wachstum angeregt werden. Im zweiten Fall (Benetzungsprinzip) wird eine flüssige Probe auf den Nährboden aufgebracht, so daß die Mikroorganismen aus der Probe in den Nährboden gelangen. Dabei ist es vorteilhaft, wenn die flüssige Probe den Nährboden gut benetzt, weil dies dazu führt, daß sie wirkungsvoll auf seiner Oberfläche verteilt wird. In der Aquaristik wird bevorzugt das Benetzungsprinzip eingesetzt.

Nach dem Benetzungsprinzip arbeitende Systeme sind so aufgebaut, daß auf einer Unterlage (meist aus Kunststoff), die als Träger wirkt, eine zweite Schicht angebracht ist. Die zweite Schicht enthält das Nährmedium. Sie ist bei bekannten Nährböden aus einem textilen Material wie z.B. Zellstoff oder Filz gebildet. Solche Materialien weisen ein offenes Volumen auf (d.h. den Teil, der nicht von ihrer Faserstruktur eingenommen wird), in welches das Nährmedium eindringen kann.

Solche bekannte Nährböden haben den Nachteil einer begrenzten Aufnahmekapazität. Grundsätzlich ergeben sich in der Praxis Probleme mit der Viskosität eines flüssig aufgebrachten Nährmediums, die dazu führen, daß das vorhandene offene Volumen des textilen Materials nicht vollständig ausgenutzt werden kann. Wählt man die Viskosität des Nährmediums hoch, ist es nicht in der Lage, in die Faserstruktur des Textilmaterials einzudringen. Wählt man sie niedriger, dringt es zwar ein, läuft jedoch hindurch und bleibt nicht gespeichert. Um das textile Material also mit der flüssigen Nährlösung beladen zu können, muß diese einen mittlere Viskosität aufweisen, was einen Kompromiß darstellt und dazu führt, daß es nicht vollständig eindringt und ein Teil des offenen Volumens ungenutzt bleibt.

EP-A-0 398 703 beschreibt ein Trägersystem, das durch einen flächigen Träger gebildet wird, und mit einer gas-durchlässigen Membran bedeckt ist. Das Närmedium wird auf die Oberfläche der Membran aufgetragen. GB-A-2 333 760 und DE-A-31 28 415 beschreiben ein flächige Träger, deren Oberflächen mit einen kanalartigen Struktur versehen sind.

Die vorliegende Erfindung stellt sich die Aufgabe, die vorstehend genannten Nachteile von bekannten Nährböden zu beseitigen und einen Nährboden zur Verfügung zu stellen, der eine hohe Aufnahmekapazität für ein flüssig aufzutragendes Nährmedium hat, wobei das Auftragen des Nährmediums einfach durchzuführen ist und keine aufwendige Produktionsanlage erfordert.

Diese Aufgabe wird gelöst, mit einem Trägersystem für ein Nährmedium gemäß Anspruch 1, einem Nährboden gemäß Anspruch 16 sowie mit den Verfahren zu deren Herstellung gemäß Anspruch 7 und Anspruch 19. Bevorzugte Ausführungsformen des Trägersystems, des Nährbodens und der Verfahren sind in den jeweiligen Unteransprüchen beschrieben.

Das erfindungsgemäße Trägersystem wird durch einen flächigen Träger gebildet, der mit einem verfestigten Polymer beschichtet ist. Das Polymer weist eine zur Aufnahme des Nährmediums dienende Struktur aus kanalartigen Hohlräumen auf, wobei die Struktur so geartet ist, daß die Hohlräume im wesentlichen parallel zur Ebene des Trägersystems verlaufen, unterhalb der Oberfläche der Polymers einseitig verschlossenen sind und mit Porenöffnungen an der Oberfläche des Polymers enden.

Die vorstehend beschriebene Struktur aus kanalartigen Hohlräumen gibt dem erfindungsgemäßen Trägersystem wesentliche Vorteile gegenüber bekannten Trägersystemen, bei denen das Nährmedium lediglich in das offene Volumen eines textilen Materials eingeführt werden kann. Die Hohlräume wirken wie ein geschlossener Sack, durch dessen Öffnung Material einführt werden kann, ohne daß es den Sack an seinem anderen Ende verlassen kann. Dies erhöht die Aufnahmekapazität für das Nährmedium wesentlich, weil die Hohlräume dauerhafte Vorratskammern für das Nährmedium bilden.

Das Beladen des Trägersystems erfolgt durch Aufbringen eines verflüssigten Nährmediums auf die Oberfläche des verfestigten Polymers. Die Viskosität des Nährmediums kann dabei relativ niedrig sein, so daß es ohne Schwierigkeiten durch die Porenöffnung in die Hohlräume eindringen kann. Da die Hohlräume an der anderen Seite geschlossen sind, kann das Nährmedium das Trägersystem nicht durchfliesen und verbleibt in den Hohlräumen. Die Viskosität des Nährmediums kann durch seine Zusammensetzung oder Temperatur beeinflußt werden. Nährmedien enthalten in der Praxis meist eine bestimmte Menge Agar, der dem Nährmedium bei Zimmertemperatur eine dickflüssige Konsistenz gibt. Beim Erwärmen auf etwa 40 bis 50 °C werden solche Lösungen (teils durch rein physikalische Erniedrigung der Viskosität, teils durch Beseitigung der Kräfte, die bei Zimmertemperatur eine gelartige Struktur bilden) relativ niederviskos und können in diesem Zustand eingesetzt werden. Läßt man die Agarlösung anschließend erkalten, stellt dies den hochviskosen Zustand bei Zimmertemperatur wieder her und begünstigt so das Verbleiben des Nährmediums in den Hohlräumen.

Als flächiger Träger eignen sich z.B. gewebte oder ungewebte textile Schichtmaterialen (Wovens, Nonwovens) aus Polymerfasern oder Schichtmaterialien auf Cellulosebasis. Bevorzugt sind flächenstabile Vliese, die bei Benetzung wenig oder nicht schrumpfen. Weiterhin bevorzugt sind thermisch gebundene Materialien, weil chemische Bindemittel unerwünschte Nährstoffquellen für die Mikroorganismen ergeben können. Der im Moment am meisten bevorzugte textile Träger ist ein flächenstabiler, thermisch gebundener Vliesstoff aus Polyethylenterephtalatfasern mit wirrer Faserstruktur mit einer Dicke im Bereich von 0,187 bis 0.193 mm, einem Flächengewicht von 90 bis 110 g/cm² und einer Luftdurchlässigkeit von 200 bis 400 dm³/(s x m²) bei 2 mbar. Ein solcher Vliesstoff wird von der Firma Freudenberg Faservliesstoffe KG, 69465 Weinheim, unter der Artikelbezeichnung FO 2413 und der Marke Viledon auf den Markt gebracht. Für dieses Material werden neben den vorgenannten Kenndaten eine Höchstzugkraft EN 29 073/03 von > 110 N/5 cm in Längsrichtung und von > 200 N/5 cm in Querrichtung angegeben. Die Höchstzugkraftdehnung EN 29 073/03 beträgt < 15 % in Längsrichtung und < 35 % in Querrichtung.

Ein bevorzugtes Polymer ist ein inkompatibles Polymerblend, wobei ein Gemisch aus Polysulfon und Polyethersulfon besonders bevorzugt ist. Besonders bevorzugt sind Polysulfon und Polyethersulfon mit jeweils 40 bis 60 Gewichtsteilen vorhanden.

Neben dem vorstehend erläuterten Trägersystem ist Gegenstand der Erfindung auch ein Verfahren zu dessen Herstellung. Dabei beschichtet man den flächigen Träger mit einer Lösung des Polymers und fällt das Polymer aus, indem man auf mindestens eine Oberfläche des beschichteten Trägers ein Fällungsmittel einwirken läßt und das Polymer einer diffusionskontrollierten Fällung unterzieht.

Bei diesen Vorgang kommt das Fällungsmittel zunächst nur mit der Oberfläche des beschichteten Trägers in Berührung, auf die man es einwirken läßt und liegt dort in sehr hoher Konzentration vor, was zu einer sehr schnellen und daher effektiven Fällung des Polymers führt. Anschließend findet ein diffusionskontrollierter Transport des Fällungsmittels durch die Polymerlösung in den Innenbereich des Trägers statt. Da der diffusionskontrollierte Transport sehr langsam erfolgt, nimmt die Konzentration des Fällungsmittels in dieser Richtung steil ab, d.h. es steht im Innenbereich nur in sehr geringer Konzentration zur Verfügung. Die Effektivität der Fällung ist somit dort wesentlich kleiner als an der Oberfläche des Trägers, auf der das Fällungsmittel mit der Polymerlösung in Berührung gebracht wurde. Es wird angenommen, daß dies zur Bildung der erfindungsgemäßen Hohlräume führt.

Geeignet zum Einsatz beim vorstehend beschriebenen Verfahren der diffusionskontrollierten Fällung sind insbesondere sog. "inkompatible Polymerblends". Darunter werden Gemische aus zwei Polymerkomponenten verstanden, die nicht miteinander mischbar sind. Nicht miteinander mischbare Polymere sind solche, deren Gemisch eine positive Mischungsenergie ΔGₘ bzw. ΔHₘ aufweist. Ein besonders geeignetes inkompatibles Polymerblend ist das bereits erwähnte Gemisch aus Polysulfon und Polyethersulfon, wobei besonders bevorzugt ein Gemisch aus Polysulfon und Polyethersulfon mit jeweils 40 bis 60 Gewichtsteilen verwendet wird.

Allgemein werden beim erfindungsgemäßen Verfahren für das Polymer aprotische und mit Wasser mischbare Lösungsmittel und als Fällungsmittel Wasser bevorzugt.

Brauchbare Lösungsmittel mit diesen Eigenschaften sind N-Methylpyrrolidon, Dimethylformamid, Dimethylsulfoxid oder Gemische davon, wobei Methylpyrrolidon zum Lösen des Gemisches aus Polysulfon und Polyethersulfon bevorzugt ist. Bevorzugt enthält die verwendete Lösung 40 bis 60 Gewichtsteile Polysulfon und 40 bis 60 Gewichtsteile Polyethersulfon, wobei die Polymerkonzentration, bezogen auf die Summe aus Polysulfon und Polyethersulfon, im Bereich von 1 bis 50 Gew.-% liegt. Nach dem Ausfällen des Polymergemisches wird das N-Methylpyrrolidon daraus bevorzugt mit Wasser ausgewaschen.

Zur Herstellung der Lösung des Polymers wird das Polymer üblicherweise in der Form von Pulver oder von Flocken eingesetzt. Um zu verhindern, daß pulver- oder flockenförmiges Material beim Einbringen in das Lösungsmittel verklumpt, empfiehlt es sich, das Material bei abgesenkter Temperatur (bis hin zu - 5 °C) vorzuquellen. Die abgesenkte Temperatur verhindert dabei eine vorzeitige Aktivierung der Oberfläche und eine Aggregation der pulver- oder flockenförmigen Teilchen. Diese können sich dann im Lösungsmittel verteilen und stehen für den weiteren Lösungsvorgang zur Verfügung. Zur Beschleunigung des Lösens empfiehlt sich gegebenenfalls eine Temperaturerhöhung auf bis zu 90 °C.

Es wurde gefunden, daß es der Bildung der erfindungsgemäßen Hohlräume förderlich ist, wenn bei Einsatz von Wasser als Fällungsmittel dieses Wasser einen Mindestgehalt an Calciumionen hat. Bevorzugt ist ein Bereich von 3,6 bis 9 mmol/l Calciumionen. Dies entspricht einer deutschen Härte von 20-50 ° Calcium. Es wird vermutet, daß durch die Anwesenheit von Calcium eine Hohlraumstruktur entsteht, wie sie für eine ionotrope Gelbildung typisch ist.

Besonders bevorzugt ist eine Ausführungsform des Verfahrens, bei dem das Beschichten des textilen Trägers mit einer Rakel durchgeführt wird. Dabei wird die Lösung des Polymers auf einer Oberfläche des textilen Trägers aufgebracht. Dann läßt man das Fällungsmittel auf die gerakelte Oberfläche des Trägers einwirken und führt die diffusionskontrollierte Fällung durch.

Das Ausfällen des Polymers ist aber nicht auf die obige Ausführungsform beschränkt. Ganz allgemein kann nicht nur eine Oberfläche des beschichteten Trägers behandelt werden, sondern auch beide Oberflächen. Hierzu kann er beispielsweise kontinuierlich oder diskontinuierlich durch ein Fällbad geführt werden. Bei beidseitiger Behandlung mit dem Fällungsmittel entsteht die Hohlraumstruktur auf beiden Oberflächen des Trägersystems.

Gegenstand der Erfindung ist auch ein Nährboden, insbesondere zum Nachweis der mikrobiologischen Belastung einer Wasserprobe, der das vorstehend beschriebene Trägersystem und ein darauf aufgebrachtes Nährmedium aufweist, wobei das Nährmedium die Struktur der kanalartigen Hohlräume zumindest teilweise ausfüllt. Bevorzugt enthält dieser Nährboden einen zur Anfärbung der Kolonien dienenden Farbindikator und weist auf der das Nährmedium tragenden Oberfläche ein zur Kolonienzählung dienendes Raster auf. Geeignete Farbindikatoren sind Nitroblau-Tetrazoliumchlorid (NBT) und Triphenyltetrazoliumchlcrid (TTC), wobei letzterer bevorzugt ist.

Gegenstand der Erfindung ist schließlich ein Verfahren zur Herstellung des Nährbodens, beim dem man eine Lösung des Nährmediums auf die die kanalartigen Hohlräume aufweisende Oberfläche des Trägerssytems aufbringt und in die Hohlräume eindringen läßt. Bei reversibel und mit geringen Wassermengen quellbaren Nährmedien kann die aufgebrachte Lösung getrocknet werden. Wenn dies nicht gegeben ist, sollte ein Trocknen wegen des damit verbundenen Wasserentzugs vermieden werden. Der Nährboden wird dann versiegelt und in dieser Form aufbewahrt.

Weitere Vorteile, Merkmale und Anwendungsmöglichkeiten der Erfindung ergeben sich aus der nachfolgenden Beschreibung von bevorzugten Ausführungsformen in Verbindung mit der Zeichnung.

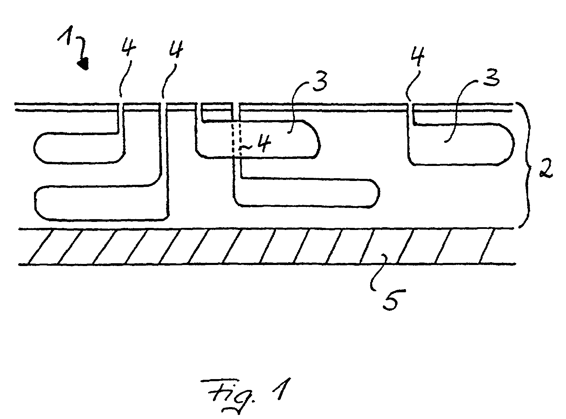

Fig. 1 ist eine schematische Darstellung des Querschnitts eines erfindungsgemäßen Trägersystems 1. Gezeigt sind der flächige Träger 5 und die Struktur aus kanalartigen Hohlräumen 3 im verfestigten Polymer 2, die im wesentlichen parallel zur Ebene des Trägersystems 1 verlaufen. Die eine Seite der Hohlräume 3 ist verschlossen. Die andere endet mit einer Porenöffnungen 4 an der Oberfläche des Polymers 2. Hierzu weisen die parallel zur Ebene des Trägersystems 1 liegenden Hohlräume 3 Bereiche auf, die ihre Lagerichtung ändern, nach oben verlaufen und dort die Porenöffnung 4 tragen. Es ist zu erkennen, daß die Hohlräume 3 in mehreren übereinander liegenden Ebenen angeordnet sind. Trotzdem endet jede Porenöffnung 4 jeweils an der Oberfläche des Polymers 2. Dies bedeutet, daß der nach oben verlaufende Bereich von weiter unten angeordneten Hohlräumen 3 länger ist.

Fig. 1 entspricht der tatsächlichen Struktur des erfindungsgemäßen Trägersystems 1 wie sie durch rasterelektronenmikroskopische Aufnahmen eines aufgerakelten und mit Wasser ausgefällten Gemisches aus Polysulfon und Polyethersulfon in N-Methylpyrrolidon ermittelt wurde. Die Porenöffnungen 5 haben einen Durchmesser von etwa 2 bis 5 µm.

Die nachfolgenden Beispiele erläutern die Erfindung weiter. Es werden folgende Abkürzungen verwendet:
- PS: Polysulfon
- PES: Polyethersulfon
- NMP: N-Methylpyrrolidon
- TTC: Triphenyltetrazoliumchlorid

### Beispiel 1 (Herstellung einer PS/PES-Lösung in NMP)

In einer Glasflasche mit 2000 ml wird 860 g NMP vorgelegt und darin 70 g PS gelöst. Das Lösen erfolgt unter Rühren mit einem Korbrührer, wobei das PS in Pulverform portionsweise mit Hilfe eines Trichters zugegeben wird. Es empfiehlt sich, neues PS jeweils erst dann zuzugeben, wenn die vorherige Portion fast vollständig gelöst ist; dies dauert jeweils etwa 30 min. Nach vollständigen Lösen des PS werden in gleicher Weise 70 g PES in Flockenform zugegeben und gelöst. Insgesamt nimmt der Vorgang etwa 23 h in Anspruch. Die entstehende Lösung hat einen Gesamtpolymergehalt von 14 Gew.-%, der zur Hälfte (d.h. zu jeweils 7 Gew.-%) durch PS und PES gebildet wird.

### Beispiel 2 (Herstellung eines erfindungsgemäßen Trägersytems)

Die Herstellung erfolgt mit dem vorstehend beschriebenen Vliesstoff FO 2413 der Firma Freudenberg Faservliesstoffe KG als flächiger Träger. Trägerplatten aus Glas (22 cm x 30 cm bzw. 30 x 40 cm) werden mit Aceton gründlich gereinigt. Der Vliesstoff wird mit geringer Übergröße auf die Größe der verwendeten Trägerplatten zugeschnitten, und zwar so, daß er über die Breite der Trägerplatten oben und unten umgeschlagen werden kann.

Ein Stück des zugeschnittenen Vliesstoffs wird auf eine Glasplatte gespannt und zur Fixierung mit jeweils zwei Klammern an den breiten Seiten befestigt. Es muß darauf geachtet werden, daß der Vliesstoff keine Falten wirft. Dann wird die bespannte Glasplatte an einer Kante angelegt, um ein Verrutschen zu verhindern.

Zur Fällung werden Kunststoffschalen eingesetzt, die etwas größer sind als die verwendete Trägerplatte und in die soviel kaltes Wasser gegeben wird, daß der beschichtete Träger anschließend in das als Fällbad dienende Wasser vollständig eintaucht. Hierzu werden etwa 1,5 bis 2 I Wasser benötigt. Der beschichtete Träger sollte jedoch nicht zu tief eintauchen, da sonst die Gefahr der Bildung von Wellen gegeben ist.

Es wird dann am oberen Rand des Vliesstoffs die gemäß Beispiel 1 hergestellte Polymerlösung in Form eines ca. 3 cm breiten Streifens aufgebracht. Dann wird eine Rakel von 200µm in die Mitte der Polymerlösung gesetzt und unter Druck von oben nach unten gezogen, wobei die Polymerlösung möglichst dünn verteilt werden sollte, jedoch ohne Bildung von Löchern oder Luftblasen.

Nach dem Rakeln läßt man die Trägerplatte mit den beschichteten Träger in das vorbereitete Fällbad gleiten, wo die Verfestigung des Polymers durch Ausfällung erfolgt. Zum Auswaschen des Lösungsmittels NMP wird das Wasser des Fällbads anschließend drei- bis viermal gewechselt. Der beschichtete Träger verbleibt wenigstens 24 h im Fällbad. Dann wird er daraus entnommen, von der Trägerplatte entfernt und zum Trocknen an Luft aufgehängt.

Es empfiehlt sich, die Rakel und alle mit der Polymerlösung in Berührung gekommenen Arbeitsgeräte sofort nach Verwendung mit Tüchern zu reinigen.

### Beispiel 3 (Herstellug eines Nährmediums)

In einer Glasflasche mit 250 I werden 1,33 g Fleischextrakt "Lab-Lemco", 1,33 g Pepton, 0,67 g Natriumchlorid und 2,00 g Agar eingewogen. Es werden 100 ml destilliertes Wasser hinzugegeben und bis zum vollständigen Lösen des Gemisches im Wasserbad erhitzt. Anschließend wird die entstandene Lösung des Nährmediums 15 min lang bei 121 °C autoklaviert.

Pro 1 ml der Lösung des Nährmediums werden 0,5 mg TTC in ein Reagenzglas eingewogen und durch Zugabe von etwa 3 ml destilliertem Wasser (steril, pH-Wert > 7) gelöst.

Der pH-Wert der Lösung des Nährmediums soll 7,3 ± 0,2 betragen. Ein solcher pH-Wert wird durch Zugabe von 0,1 N NaOH eingestellt, nachdem die Lösung auf 50 °C abgekühlt ist. Die Zugabe erfolgt mit einer Pipette unter ständiger Temperaturkontrolle. Nach dem Einstellen des pH-Werts wird die Lösung von TTC zur Lösung des Nährmediums gegeben und gut damit vermischt. Die Temperatur der Lösung des Nährmediums sollte dabei ≤ 45 °C betragen.

Das Nährmedium wird anschließend sofort gegossen. Sollte es bis dahin bereits erstarrt sein, muß es zur Verflüssigung im Wasserbad bei 45 °C erwärmt werden.

### Beispiel 4 (Herstellung eines erfindungsgemäßen Nährbodens)

Zunächst wird das gemäß Beispiel 3 hergestellte Trägersystem in die gewünschte Form gebracht. Dies kann bei einer rechteckigen Form durch Zuschneiden des beschichteten Trägers oder bei einer runden Form durch Ausstanzen mit einem Locheisen erfolgen. Je nach Bedürfnissen kann die Form so geartet sein, daß ein Festhaltegriff vorgesehen ist, mit dem das Trägersystem gehandhabt werden kann, ohne daß die später mit der Probe in Kontakt kommenden Bereiche berührt werden.

Nach der Formgebung wird der beschichtete Träger mit der beschichteten Seite nach oben auf eine Unterlage aus Glas gelegt. Dann wird das gemäß Beispiel 3 hergestellte Nährmedium durch Erwärmen auf etwa 40 °C verflüssigt und auf den beschichteten Träger gegossen. Es sollte eine Schicht mit einer Dicke von etwa 0,5 mm entstehen. Der so hergestellte erfindungsgemäße Nährboden wird nicht getrocknet, sondern in eine Umgebung gebracht, in der er möglichst wenig Wasser verliert, um die Quellbarkeit des Nährmediums zu erhalten. Deshalb wird er bis zur Weiterverwendung verschlossen aufbewahrt (z.B. in einer Petrischale mit Deckel) und später zum Versand in Folien eingeschweißt.

### Beispiel 5 (Einsatz eines erfindungsgemäßen Nährbodens)

Zur Erprobung des gemäß Beispiel 4 hergestellten erfindungsgemäßen Nährbodens wurde ein Aquariumwasser mit folgenden Wasserparametern eingesetzt:

| | | |
|---|---|---|
| Temperatur | 24 °C | |
| pH-Wert | 7,3 | (MN-Schnelltest) |
| Wasserhärte | 15 °d (267 mg/l bzw. 2,67 mmol/l Calciumcarbonat) | (Fluka-Schnelltest) |
| Nitratgehalt | 50 mg/l; kein Nitrit | (Merck-Schnelltest) |
| Sauerstoffgehalt | 8,0 mg/l (95 % Sättigung) | |
| Leitfähigkeit | 610 µS/cm | |

Die erfindungsgemäßen Nährboden wurden in Petrischalen mit dem vorgenannten Aquariumswasser übergossen und 24 Stunden lang bei einer Temperatur von 28 °C inkubiert. Es zeigte sich eine gute Koloniebildung. Die Kolonien waren rot angefärbt und konnten gut ausgezählt werden.

### Bezugszeichenliste

- 1: Trägersystem
- 2: Polymer
- 3: Hohlräume
- 4: Porenöffnung
- 5: flächiger Träger

## Patentansprüche

1. Trägersystem (1) für ein Nährmedium,
**dadurch gekennzeichnet,**
**daß** das Trägersystem (1) durch einen flächigen Träger (5) gebildet ist, der mit einem verfestigten Polymer (2) beschichtet ist,
wobei das Polymer (2) eine zur Aufnahme des Nährmediums dienende Struktur aus kanalartigen Hohlräumen (3) aufweist, die im wesentlichen parallel zur Ebene des Trägersystems (1) verlaufen, unterhalb der Oberfläche der Polymers (2) einseitig verschlossenen sind und mit Porenöffnungen (4) an der Oberfläche des Polymers (2) enden.

2. Trägersystem nach Anspruch 1, **dadurch gekennzeichnet, daß** der flächige Träger (5) ein gewebtes oder ungewebtes textiles Schichtmaterial aus Polymerfasern oder ein Schichtmaterial auf Cellulosebasis ist.

3. Trägersystem nach Anspruch 2, **dadurch gekennzeichnet, daß** der flächige Träger (5) ein flächenstabiler, thermisch gebundener Vliesstoff aus Polyethylenterephtalatfasern mit wirrer Fadenstruktur ist, wobei der Viesstoff im nicht imprägnierten Zustand eine Dicke von 0,187 bis 0,193 mm, ein Flächengewicht von 90 bis 110 g/cm² und eine Luftdurchlässigkeit von 200 bis 400 dm³/(s x m²) bei 2 mbar hat.

4. Trägersystem nach einem der Ansprüche 1 bis 3, **dadurch gekennzeichnet, daß** das Polymer (2) ein inkompatibles Polymerblend ist.

5. Trägersystem nach Anspruch 4, **dadurch gekennzeichnet, daß** das inkompatible Polymerblend ein Gemisch aus Polysulfon und Polyethersulfon ist.

6. Trägersystem nach Anspruch 5, **dadurch gekennzeichnet, daß** das Gemisch 40 bis 60 Gewichtsteile Polysulfon und 40 bis 60 Gewichtsteile Polyethersulfon enthält.

7. Verfahren zur Herstellung des Trägersystems für ein Nährmedium gemäß einem der vorhergehenden Ansprüche,
**dadurch gekennzeichnet,**
**daß** man einen flächigen Träger mit einer Lösung eines Polymers beschichtet und das Polymer ausfällt, indem man auf mindestens eine Oberfläche des beschichteten Trägers ein Fällungsmittel einwirken läßt und das Polymer einer diffusionskontrollierten Fällung unterzieht.

8. Verfahren nach Anspruch 7, **dadurch gekennzeichnet, daß** man als Lösungsmittel für das Polymer ein aprotisches und mit Wasser mischbares Lösungsmittel und als Fällungsmittel Wasser einsetzt.

9. Verfahren nach Anspruch 8, **dadurch gekennzeichnet, daß** man als Lösungsmittel N-Methylpyrrolidon, Dimethylformamid, Dimethylsulfoxid oder Gemische davon einsetzt.

10. Verfahren nach Anspruch 8 oder 9, **dadurch gekennzeichnet, daß** das Wasser 3,6 bis 9 mmol/l Calciumionen enthält (20-50 °d Calcium).

11. Verfahren nach einem der Ansprüche 7 bis 10, **dadurch gekennzeichnet, daß** das Polymer ein inkompatibles Polymerblend ist.

12. Verfahren nach Anspruch 11, **dadurch gekennzeichnet, daß** das inkompatible Polymerblend ein Gemisch aus Polysulfon und Polyethersulfon ist.

13. Verfahren nach Anspruch 12, **dadurch gekennzeichnet, daß** man 40 bis 60 Gewichtsteile Polysulfon und 40 bis 60 Gewichtsteile Polyethersulfon einsetzt, wobei die Polymerkonzentration, bezogen auf die Summe aus Polysulfon und Polyethersulfon, im Bereich von 1 bis 50 Gew.-% liegt.

14. Verfahren nach einem der Ansprüche 8 bis 13, **dadurch gekennzeichnet, daß** man nach dem Ausfällen des Polymergemisches das Lösungsmittel daraus mit Wasser auswäscht.

15. Verfahren nach einem der Ansprüche 7 bis 14, **dadurch gekennzeichnet, daß** man das Beschichten des flächigen Trägers durchführt, indem man die Lösung auf einer Oberfläche des flächigen Trägers mit einer Rakel aufbringt und das Fällungsmittel auf die gerakelte Oberfläche des Trägers einwirken läßt.

16. Nährboden, insbesondere zum Nachweis der mikrobiologischen Belastung einer Wasserprobe, aufweisend ein Trägersystem gemäß einem der vorhergehenden Ansprüche und ein darauf aufgebrachtes Nährmedium,
**dadurch gekennzeichnet,**
**daß** das Nährmedium die Struktur der kanalartigen Hohlräume zumindest teilweise ausfüllt.

17. Nährboden nach Anspruch 16, **dadurch gekennzeichnet, daß** die das Nährmedium tragende Oberfläche des Nährbodens ein zur Kolonienzählung dienendes Raster aufweist.

18. Nährboden nach Anspruch 16 oder 17, **dadurch gekennzeichnet, daß** das Nährmedium einen zur Anfärbung der Kolonien dienenden Farbindikator enthält.

19. Verfahren zur Herstellung des Nährbodens gemäß einem der Ansprüche 16 bis 18,
**dadurch gekennzeichnet,**
**daß** man eine Lösung des Nährmediums auf die die kanalartigen Hohlräume aufweisende Oberfläche des Trägersystems gemäß einem der Ansprüche 1 bis 6 aufbringt und in die Hohlräume eindringen läßt.

## Claims

1. A carrier system (1) for a culture medium,
**characterised in that** the carrier system (1) is formed by a flat carrier (5) which is coated with a hardened polymer (2),
wherein the polymer (2) has a structure serving to receive the culture medium and comprising passage-like cavities (3) which extend substantially parallel to the plane of the carrier system (1), are closed at one side beneath the surface of the polymer (2) and terminate with pore openings (4) at the surface of the polymer (2).

2. A carrier system according to claim 1 **characterised in that** the flat carrier (5) is a woven or non-woven textile layer material comprising polymer fibres or a cellulose-base layer material.

3. A carrier system according to claim 2 **characterised in that** the flat carrier (5) is a surface-stable, thermally bound fleece substance comprising polyethylene terephthalate fibres with a tangled thread structure, wherein the fleece substance in the non-impregnated condition is of a thickness of from 0.187 to 0.193 mm, is of a weight in relation to surface area of from 90 to 110 g/cm², and has an air permeability of from 200 to 400 dm³/(s x m²) at 2 mbar.

4. A carrier system according to one of claims 1 to 3 **characterised in that** the polymer (2) is an incompatible polymer blend.

5. A carrier system according to claim 4 **characterised in that** the incompatible polymer blend is a mix of polysulphone and polyethersulphone.

6. A carrier system according to claim 5 **characterised in that** the mix contains from 40 to 60 parts by weight of polysulphone and from 40 to 60 parts by weight of polyethersulphone.

7. A process for producing the carrier system for a culture medium according to one of the preceding claims **characterised in that** a flat carrier is coated with a solution of a polymer and the polymer is precipitated by causing a precipitation agent to act on at least one surface of the coated carrier and subjecting the polymer to diffusion-controlled precipitation.

8. A process according to claim 7 **characterised in that** the solvent used for the polymer is an aprotic, water-miscible solvent and the precipitation agent used is water.

9. A process according to claim 8 **characterised in that** the solvent used is N-methylpyrrolidone, dimethylformamide, dimethylsulphoxide or mixtures thereof.

10. A process according to claim 8 or claim 9 **characterised in that** the water contains from 3.6 to 9 mmol/l of calcium irons (20-50°d calcium).

11. A process according to one of claims 7 to 10 **characterised in that** the polymer is an incompatible polymer blend.

12. A process according to claim 11 **characterised in that** the incompatible polymer blend is a mix of polysulphone and polyethersulphone.

13. A process according to claim 12 **characterised in that** from 40 to 60 parts by weight of polysulphone and from 40 to 60 parts by weight of polyethersulphone are used, wherein the polymer concentration in relation to the sum of polysulphone and polyethersulphone is in the range of from 1 to 50% by weight.

14. A process according to one of claims 8 to 13 **characterised in that** after precipitation of the polymer mix the solvent is washed out of same with water.

15. A process according to one of claims 7 to 14 **characterised in that** the operation of coating the flat carrier is effected by applying the solution on a surface of the flat carrier with a squeegee and causing the precipitation agent to act on the squeegeed surface of the carrier.

16. A culture substrate, in particular for detection of the microbiological loading of a water sample, having a carrier system according to one of the preceding claims and a culture medium applied thereto, **characterised in that** the culture medium at least partially fills the structure of the passage-like cavities.

17. A culture substrate according to claim 16 **characterised in that** the surface of the culture substrate, which carries the culture medium, has a grid for colony counting.

18. A culture substrate according to claim 16 or claim 17
**characterised in that** the culture medium includes a colour indicator serving to colour the colonies.

19. A process for producing the culture substrate according to one of claims 16 to 18 **characterised in that** a solution of the culture medium is applied to the surface of the carrier system according to one of claims 1 to 6, which surface has the passage-like cavities, and the solution is caused to penetrate into the cavities.

## Revendications

1. Système de support (1) pour un milieu nutritif,
**caractérisé en ce**
**que** le système de support (1) est formé d'un support plat (5), qui est revêtu d'un polymère (2) consolidé, le polymère (2) présentant une structure servant à recevoir le milieu nutritif, formé d'espaces vides (3) en forme de canaux qui sont disposés sensiblement parallèlement au plan du système de support (1), sont fermés d'un côté au-dessous de la surface du polymère (2) et finissent par des ouvertures de pores (4) à la surface du polymère (2).

2. Système de support selon la revendication 1, **caractérisé en ce que** le support plat (5) est une matière textile en couches, tissée ou non tissée, en fibres polymères ou une matière en couches à base de cellulose.

3. Système de support selon la revendication 2, **caractérisé en ce que** le support plat (5) est un tissu non-tissé à surface stable, lié thermiquement, en fibres de téréphtalate de polyéthylène ayant une structure de fils emmêlés, le tissu non-tissé à l'état non imprégné ayant une épaisseur de 0,187 à 0,193 mm, une masse surfacique de 90 à 110 g/cm² et une perméabilité à l'air de 200 à 400 dm³/(s x m²) à 2 mbar.

4. Système de support selon l'une des revendications 1 à 3, **caractérisé en ce que** le polymère (2) est un mélange polymère incompatible.

5. Système de support selon la revendication 4, **caractérisé en ce que** le mélange polymère incompatible est un mélange de polysulfone et de polyéthersulfone.

6. Système de support selon la revendication 5, **caractérisé en ce que** le mélange contient 40 à 60 parties en poids de polysulfone et 40 à 60 parties en poids de polyéthersulfone.

7. Procédé pour la fabrication du système de support pour un milieu nutritif selon l'une des revendications précédentes,
**caractérisé en ce**
**qu'**on revêt un support plat d'une solution d'un polymère et on précipite le polymère en faisant agir un agent de précipitation sur au moins une surface du support revêtu, et en faisant subir au polymère une précipitation contrôlée par diffusion.

8. Procédé selon la revendication 7, **caractérisé en ce qu'**on utilise comme solvant pour le polymère un solvant aprotique miscible à l'eau et de l'eau comme agent de précipitation.

9. Procédé selon la revendication 8, **caractérisé en ce qu'**on utilise comme solvant de la N-méthylpyrrolidone, du diméthylformamide, du diméthylsulfoxyde ou des mélanges de ceux-ci.

10. Procédé selon l'une des revendications 8 et 9, **caractérisé en ce que** l'eau contient de 3,6 à 9 mmol/l d'ions calcium (20-50 °d calcium).

11. Procédé selon l'une des revendications 7 à 10, **caractérisé en ce que** le polymère est un mélange polymère incompatible.

12. Procédé selon la revendication 11, **caractérisé en ce que** le mélange polymère incompatible est un mélange de polysulfone et de polyéthersulfone.

13. Procédé selon la revendication 12, **caractérisé en ce qu'**on utilise 40 à 60 parties en poids de polysulfone et 40 à 60 parties en poids de polyéthersulfone, la concentration des polymères, par rapport à la somme de la polysulfone et de la polyéthersulfone, étant comprise entre 1 et 50 % en poids.

14. Procédé selon l'une des revendications 8 à 13, **caractérisé en ce que**, après la précipitation du mélange polymère, on élimine le solvant de celui-ci par lavage avec de l'eau.

15. Procédé selon l'une des revendications 7 à 14, **caractérisé en ce qu'**on effectue l'enduction du support plat en appliquant la solution sur une surface du support plat, au moyen d'une racle, et en faisant agir l'agent de précipitation sur la surface du support enduite à la racle.

16. Milieu de culture, en particulier pour la détection de la charge microbiologique d'un échantillon d'eau, présentant un système de support selon l'une des revendications précédentes et un milieu nutritif appliqué sur celui-ci,
**caractérisé en ce**
**que** le milieu nutritif remplit au moins partiellement la structure des espaces creux en forme de canaux.

17. Milieu de culture selon la revendication 16, **caractérisé en ce que** la surface du milieu de culture portant le milieu nutritif présente une grille servant à compter des colonies.

18. Milieu de culture selon l'une des revendications 16 et 17, **caractérisé en ce que** le milieu nutritif contient un indicateur coloré servant à la coloration des colonies.

19. Procédé pour la fabrication du milieu de culture selon l'une des revendications 16 à 18,
**caractérisé en ce**
**qu'**on applique une solution du milieu nutritif sur la surface, présentant les espaces creux en forme de canaux, du système de support selon l'une des revendications 1 à 6 et la laisse pénétrer dans les espaces creux.
